# EUROPÄISCHE PATENTSCHRIFT

(11) **EP 2 306 988 B1**
(45) Veröffentlichungstag und Bekanntmachung des Hinweises auf die Patenterteilung: **09.01.2013**
(21) Anmeldenummer: 09800023.5
(22) Anmeldetag: 02.07.2009
(51) Int. Cl.: A61K 9/46, A61K 31/58

(54) **PHARMAZEUTISCHE FORMULIERUNG ZUR BEHANDLUNG DES OBEREN VERDAUUNGSTRAKTES**
PHARMACEUTICAL FORMULATION FOR TREATING THE UPPER DIGESTIVE TRACT
FORMULATION PHARMACEUTIQUE POUR LE TRAITEMENT DU TRACTUS DIGESTIF SUPÉRIEUR

(30) Priorität: 21.07.2008 EP 08013091
(43) Veröffentlichungstag der Anmeldung: 13.04.2011
(73) Patentinhaber: Dr. Falk Pharma GmbH, 79041 Freiburg (DE)
(72) Erfinder: WILHELM, Rudolf, 76476 Bischweier (DE); PRÖLS, Markus, 79100 Freiburg/Breisgau (DE)
(74) Vertreter: Keller, Günter
(86) Internationale Anmeldenummer: PCT/EP2009/058320
(87) Internationale Veröffentlichungsnummer: WO 2010/009961

(56) Entgegenhaltungen:
- EP-B- 0 720 473
- WO-A2-2009/000473
- US-A1- 2006 013 873
- ELAD SHARON ET AL: "Budesonide: A novel treatment for oral chronic graft versus host disease." ORAL SURGERY ORAL MEDICINE ORAL PATHOLOGY ORAL RADIOLOGY AND ENDODONTICS, Bd. 95, Nr. 3, März 2003 (2003-03), Seiten 308-311, XP002504484 ISSN: 1079-2104 in der Anmeldung erwähnt
- SPENCER C M ET AL: "BUDESONIDE A REVIEW OF ITS PHARMACOLOGICAL PROPERTIES AND THERAPEUTIC EFFICACYIN INFLAMMATORY BOWEL DISEASE" DRUGS, ADIS INTERNATIONAL LTD, Bd. 50, Nr. 5, 1. Januar 1995 (1995-01-01), Seiten 854-872, XP001007442 ISSN: 0012-6667

## Beschreibung

Die vorliegende Erfindung betrifft eine pharmazeutische Formulierung, nämlich eine Brausetablette, zur Behandlung des oberen Verdauungstraktes, die als pharmazeutischen Wirkstoff Budesonid oder ein pharmazeutisch verträgliches Salz oder Derivat davon umfasst.

Budesonid enthaltende pharmazeutische Formulierungen zur oralen Anwendung mit kontrollierter Freisetzung im Intestinaltrakt sind aus EP-A-0 720 473 bekannt.

Diese Formulierungen werden schon seit längerem zur Behandlung intestinaler Erkrankungen, wie beispielsweise Morbus Crohn eingesetzt, sind wirksam (Bar-Meir, Gastroenterology, 1998, S. 835-840) und haben ein verbessertes Sicherheitsprofil (Andus, Digestive Diseases and Sciences, 1. Februar 2003, S. 373-378).

Allgemein bekannt ist die Verwendung von Glucocorticoiden, insbesondere Budesonid, zur Behandlung von Erkrankungen, die mit Entzündungsprozessen einhergehen. Zu diesen Erkrankungen zählen auch solche der Mundhöhle, des Pharynx und der Speiseröhre. Der Wirkstoff Budesonid wurde z.B. bei GVHD (graft versus host disease) erfolgreich eingesetzt (Elad et al., Oral Surg: Oral Med. Oral Pathol. and Radial Endod. 2003, 95, S. 308-311).

Die WO03/06629 beschreibt Lutschtabletten, enthaltend einen Wirkstoffkern, der mit einem wasserlöslichen Polysaccharid (Gellum Gum) umhüllt ist. Der Wirkstoff ist innerhalb des Kerns und nicht mit der Hülle durchmischt. Als mögliche Wirkstoffe werden Glucocorticoide, wie beispielsweise Budesonid, genannt. Die Tablette soll zur buccalen Anwendung geeignet sein.

Für die Behandlung von entzündlichen Prozessen in der Mundhöhle ist die Bereitstellung einer Arzneiform gewünscht, die reproduzierbar, als gebrauchsfertige Formulierung, genügend hohe Wirkstoffkonzentrationen am Entzündungsort freisetzt und eine lokale Wirkung zeigt.

Bekannt ist die direkte Anwendung des Wirkstoffes, nach Zermörsern von magensaftresistenten Kapseln zur Behandlung der GVHD. Elad et al. (Oral Surgery Oral Medicine Oral Pathology, 2003, SS 308-311) beschreiben eine Behandlung für oral manifestierte "chronic graft versus host disease"-Erkrankungen, die häufig nach der Transplantation von hämatopoetischen Stammzellen auftreten. Eingesetzt wird eine Budesonid-haltige Mundspülung. Diese im Stand der Technik genannte direkte Anwendung des Wirkstoffes hat verschiedene Nachteile, wie beispielsweise keine gebrauchsfertige, industriell herstellbare Formulierung mit reproduzierbarer Dosierung zu sein. Das Zermörsern von Tabletten hat auch den Nachteil, keine gleichmäßigen hohen Wirkstoffkonzentrationen am Ort der Entzündung bereitzustellen.

Die nicht vorveröffentlichte WO 2009/000473 beschreibt Brausetabletten, die aufgelöst in einer geeigneten physiologischen Lösung inhaliert werden können. Diese Brausetabletten können Corticosteroide, unter anderem Budesonid, zur Prophylaxe und Therapie von Symptomen von Asthma durch Inhalation einer nach Auflösung der Brausetabletten erhaltenen Lösung enthalten.

Die spezielle Tablettenformulierung dient der buccalen Anwendung, vor allem mit dem Ziel, einen Wirkstoff buccal in den systemischen Kreislauf aufzunehmen. Eine Benetzung des gesamten Bereiches der Mundhöhle mit gelöstem Wirkstoff zur lokalen Anwendung ist nicht möglich.

Die hohe Instabilität von Budesonid in gelöster Form schließt die Herstellung einer gelösten Arzneimittelpräparation, die Budesonid enthält, aus. Eine solche Budesonidlösung wäre über einen längeren Zeitraum nicht ohne genaue pH-Einstellung, sowie den Zusatz von Konservierungsmitteln und weitere Stabilisatoren stabil.

Es ist eine Aufgabe der vorliegenden Erfindung, eine oral verabreichbare pharmazeutische Formulierung bereit zu stellen, die die genannten Nachteile nicht mehr aufweist.

Erfindungsgemäß wird daher eine Brausetablette enthaltend Budesonid zur Zubereitung einer oral verabreichbaren Mundspüllösung Zur Anwerdung in der Behandlung von entzündlichen Veränderungen des oberen Verdauungstraktes zur Verfügung gestellt, die eine schnelle, verbesserte Löslichkeit für Budesonid bei der Zubereitung als Mundspüllösung zeigt, zu einer hohen lokalen Wirkstoffkonzentration führt und zudem eine sichere, nebenwirkungsarme Anwendung über einen längeren Zeitraum ermöglicht. Außerdem ermöglicht die erfindungsgemäße Brausetablette eine stabile Lagerung und einfache Handhabung.

Es wurde festgestellt, dass eine speziell zubereitete Mundspüllösung, hergestellt aus einer Brausetablette, für die Behandlung besonders geeignet ist. Im Gegensatz zu einer Tablette, kann eine solche Mundspüllösung in der gesamten Mundhöhle, sowie im Pharynx und im Ösophagus gleichermaßen angewendet werden, da der Wirkstoff in hohen Anteilen in gelöster Form vorliegt. Die Brausetablette bietet zudem große Vorteile in Bezug auf Langzeitstabilität der Arzneiform bis zur Anwendung, eine einfache und genaue Dosierung.

Ebenso bietet eine Brausetablette erhebliche Vorteile bezogen auf die Anbruchstabilität der Darreichungsform.

Die erfindungsgemäße Brausetablette zur Zubereitung einer oral verabreichbaren Mundspüllösung zur Anwendung in der Behandlung von entzündlichen Veränderungen des oberen Verdauungstraktes enthält Budesonid. Der IUPAC-Name von Budesonid lautet: 16,17-(butylidenebis(oxy))-11,21-dihydroxy-,(11-β,16-α)-pregna-1,4-dien-3,20-dion. In bevorzugter Ausführungsform ist 0,1 bis 10 mg Budesonid pro Brausetablette enthalten. Besonders bevorzugt ist Budesonid in einer Menge von 1 mg - 5 mg und ganz besonders bevorzugt in einer Menge von etwa 3 mg pro Brausetablette vorhanden.

Das in der Brausetablette eingesetzte Budesonid muss die Qualitäts- und Reinheitsanforderungen für eine pharmazeutische Formulierung erfüllen. Bevorzugt wird mikronisiertes Budesonid eingesetzt. Für die Lösegeschwindigkeit und die Resorbierbarkeit spielt die Partikelgröße der Budesonidteilchen eine wesentliche Rolle. Bevorzugt wird Budesonid eingesetzt, bei dem die Teilchengrößeverteilung so eingestellt ist, dass wenigstens 90 % der Teilchen einen Teilchendurchmesser von weniger als 20 µm, bevorzugt weniger als 10 µm aufweisen. In einer besonders bevorzugten Ausführungsform weisen 100 % der Teilchen einen Durchmesser kleiner als 10 µm, 95 % der Teilchen einen Durchmesser kleiner als 5 µm und 80 % der Teilchen einen Durchmesser kleiner als 3 µm aus. Der Teilchendurchmesser wird mit üblichen Meßmethoden bestimmt.

Um die Löslichkeit des Budesonids in der Mundspüllösung, die aus der erfindungsgemäßen Brausetablette hergestellt wird, zu erhöhen, enthält die erfindungsgemäße Brausetablette bevorzugt Polyvinylpyrrolidon in einer Konzentration von 0,5 bis 10 Gew.-%, besonders bevorzugt in einer Menge zwischen 1,0 und 3,0 Gew.-%, jeweils bezogen auf das Gesamtgewicht der fertigen Brausetablette.

Bei Polyvinylpyrrolidon handelt es sich um ein Polymerisationsprodukt des Vinylpyrrolidons. Kommerziell erhältlich sind eine Reihe von Fraktionen mit unterschiedlichen Molekülgrößen bzw. Molekülkettenlängen. Das Molekülmassenspektrum reicht von 10.000 bis 350.000. Bevorzugt eingesetzt wird Polyvinylpyrrolidon mit einer Molekülgröße zwischen etwa 15.000 und 150.000. Eine besondere Eigenschaft der Polyvinylpyrrolidone ist die gute Löslichkeit sowohl in Wasser, wie auch in polaren organischen Lösungsmitteln, wie Alkoholen oder Glycerin.

Weiterhin enthält die erfindungsgemäße Brausetablette bevorzugt noch einen anderen Solubilisator bzw. Emulgator, nämlich Docusat-Natrium (Natriumdioctylsulphosuccinat) in einer Konzentration von 0,1 ‰ - 5 ‰, bevorzugt 0,2 ‰ - 2,0 ‰, wobei sich die Angaben auf das Gewicht der fertigen Brausetablette beziehen.

In einer ganz besonders bevorzugten Ausführungsform enthält die erfindungsgemäße Brausetablette sowohl Polyvinylpyrrolidon wie auch Docusat-Natrium

Damit sich die erfindungsgemäße Brausetablette nach Kontakt mit Wasser gut auflöst, enthält sie eine Brausemischung umfassend eine pharmazeutisch annehmbare Säure in fester Form und eine Carbonat und/oder Hydrogencarbonat enthaltende Verbindung.

Als pharmazeutisch annehmbare Säure wird bei der erfindungsgemäßen Brausetablette eine solche Säure verwendet, die in fester Form verfügbar ist, gesundheitlich unbedenklich ist und keinen unangenehmen Geschmack hervorruft. Die bevorzugt eingesetzte Säure ist Zitronensäure. Zusätzlich beinhaltet die Brausemischung eine Carbonat und/oder Hydrogencarbonat enthaltende Verbindung, die durch Kontakt mit Säure Kohlenstoffdioxid freisetzt, wodurch sich die Brausetablette auflöst. Hierbei handelt es sich bevorzugt um Natriumcarbonat oder Natriumhydrogencarbonat.

In einer bevorzugten Ausführungsform beinhaltet die erfindungsgemäße Brausetablette auch ein Mittel, das einen kühlenden Effekt (cooling effect) im Mund bewirkt. Hierbei handelt es sich in bevorzugter Ausführungsform um Butanamid (N-2,3-Trimethyl-2-isopropylbutanamid). Dieses Mittel wird bevorzugt in einer Menge von 0,1 - 1,0, besonders bevorzugt in einer Menge zwischen 0,3 und 0,8 Gew.-%, bezogen auf die fertige Brausetablette, eingesetzt.

Die erfindungsgemäße Brausetablette wird zur Zubereitung einer oral verabreichbaren Mundspüllösung zur Anwendung in der Behandlung von entzündlichen Veränderungen des oberen Verdauungstraktes verwendet.

Bei der Anwendung wird die Brausetablette in einer bestimmten Menge einer Flüssigkeit, bevorzugt Wasser, aufgelöst, wobei die für die Auflösung der Brausetablette erforderliche Wassermenge zwischen 5 und 20 ml, bevorzugt zwischen 5 und 15 ml, und besonders bevorzugt etwa 10 ml Wasser beträgt.

Bei den entzündlichen Veränderungen des oberen Verdauungstraktes, die mit der durch die Brausetablette herstellbare Mundspüllösung behandelt werden, handelt es sich bevorzugt um entzündliche Veränderungen des oberen Verdauungstraktes im Bereich der Mundhöhle und/oder des Pharynx. In üblicher Anwendungsweise wird die Mundspüllösung durch Auflösung der Brausetablette in Wasser hergestellt und dann wird mit dieser Mundspüllösung eine vorbestimmte Zeit, die zwischen 2 und 15 Minuten, bevorzugt bei etwa 10 Minuten, liegen kann, gegurgelt und der Mund gespült. Anschließend wird die Mundspüllösung nicht geschluckt, sondern ausgespuckt.

Die entzündlichen Veränderungen des oberen Verdauungstraktes sind bevorzugt nicht infektiöse Entzündungen. Sie können durch verschiedene Ursachen, beispielsweise Strahlentherapie, Organtransplantation und/oder Chemotherapie hervorgerufen werden. In bevorzugter Ausführungsform handelt es sich bei den entzündlichen Veränderungen des oberen Verdauungstraktes um Mukositis, einer Autoimmunerkrankung der Mundhöhle, Morbus Crohn im oberen Verdauungstrakt sowie eosinophile Ösophagitis.
*Figur 1* zeigt einen Vergleich der in Wasser freigesetzten Menge an Budesonid aus unterschiedlichen Brausetablettenrezepturen. Angegeben ist auch die Menge an freigesetztem Budesonid, das aus zermörserten, Magensaft-resistenten Kapseln, aufgelöst in Wasser, freigesetzt werden kann.
*Figur 2* zeigt Budesonid Plasma-Konzentrations-Zeit Kurven von 7 Patienten mit chronisch aktiver oraler Graft-Versus-Host Disease nach der Gabe von 3 verschiedenen Behandlungen mit Budesonid:
   R = per os Gabe von 10 ml wässriger Lösung mit 3 mg Budesonid.
   SD1 = 10 ml wässrige Lösung mit 3 mg Budesonid als Mundspüllösung für 10 Minuten.
   MD1 = 7 Tage lange Mehrfachdosierung einer 10 ml wässrigen Lösung mit 3 mg Budesonid, 3x täglich in einer Tagesdosis von 9 mg Budesonid als Mundspüllösung jeweils für 10 Minuten. Die letzte Gabe erfolgte morgens am 7. Tag. Die Kurven sind als Mittelwert ± Standardabweichung dargestellt.

Bevorzugte Ausführungsformen der vorliegenden Erfindung werden durch die nachfolgenden Beispiele verdeutlicht.

### Beispiel 1

Überraschenderweise wurde gefunden, dass die Löslichkeit von Budesonid-Brausetabletten durch den Zusatz von Polyvinylpyrrolidon (PVP) und Docusat-Natrium deutlich erhöht wird. Die Zusammensetzung verschiedener Brausetabletten ist in Tabelle 1 dargestellt. Die Zusammensetzung mit der Bezeichnung G0397X414 wurde näher untersucht und stellte sich in den späteren Untersuchungen als besonders geeignet heraus.

Die folgende Tabelle 1 fasst die Rezepturen der getesteten Brausetabletten zusammen. Die Zusammensetzung variiert dabei lediglich in den Bestandteilen Povidon K25 (Polyvinylpyrrolidon) und Docusat-Natrium, die zur Verbesserung der Löslichkeit von Budesonid variiert wurden. Geringfügige Unterschiede in den einzelnen Rezepturen wurden durch Mannitol ausgeglichen.

**Tab. 1 Zusammensetzung der Brausetabletten-Rezepturen**

| **Zusammensetzung [mg]** | | | | | | |
|---|---|---|---|---|---|---|
| **Schritt 1: Granulierung** | | | | | | |
| Budesonid | 3 | 3 | 3 | 3 | 3 | 3 |
| Natriumdihydrogencitrat | 67 | 67 | 67 | 67 | 67 | 67 |
| Dinatriumhydrogencitrat | 15 | 15 | 15 | 15 | 15 | 15 |
| Natriumhyrogencarbonat | 45 | 45 | 45 | 45 | 45 | 45 |
| Povidon K25 (PVP) | 2 | 2 | --- | --- | 4 | 4 |
| Docusat-Natrium | 0,05 | --- | 0,05 | --- | --- | --- |
| Aspartam | 1 | 1 | 1 | 1 | 1 | 1 |
| Granulat | 133,05 | 133 | 131,05 | 131 | 135 | 135 |

| **Schritt 2: Endmischung** | | | | | | |
|---|---|---|---|---|---|---|
| Povidon K25 (PVP) | --- | --- | --- | --- | --- | 3 |
| Mannitol | 5,95 | 6 | 7,95 | 8 | 4 | 4 |
| Macrogol 6000 | 5 | 5 | 5 | 5 | 5 | 5 |
| Butanamid¹ | 0,9 | 0,9 | 0,9 | 0,9 | 0,9 | 0,9 |
| Magnesiumstearat | 0,1 | 0,1 | 0,1 | 0,1 | 0,1 | 0,1 |
| Endmischung | 145 | 145 | 145 | 145 | 145 | 148 |
| **Brausetablette (Code)** | **G0397X414** | **G0397X415** | **G0397X416** | **G0397X417** | **G0397X418** | **G0397X419** |

| | | | | | | |
|---|---|---|---|---|---|---|
| ¹ Chemische Bezeichnung: N-2,3-Trimethyl-2-isopropylbutanamid | | | | | | |

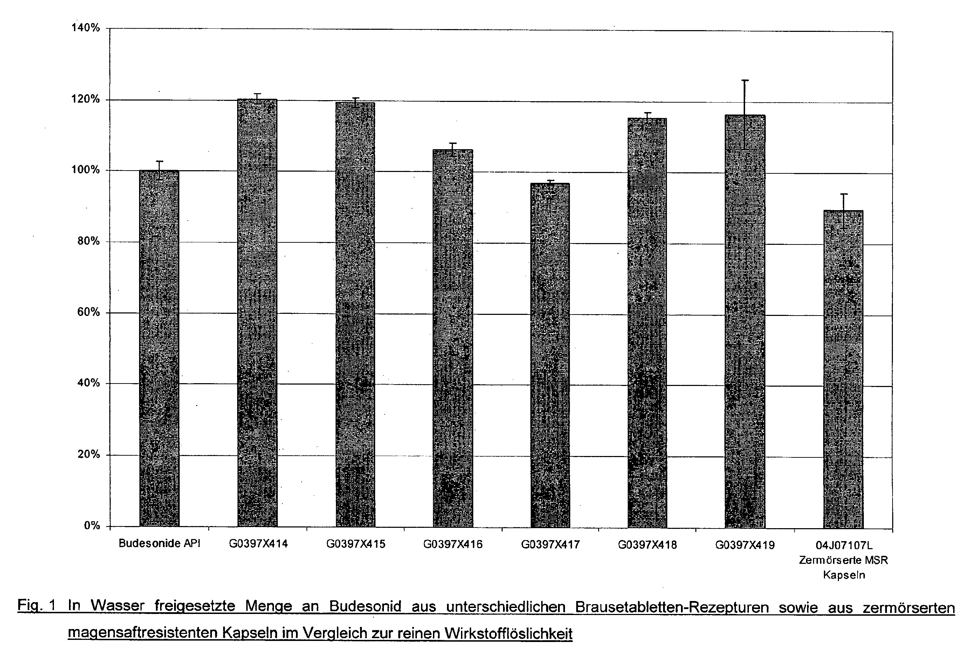

Die Bestimmung der Löslichkeit von Budesonid aus den einzelnen Brausetabletten erfolgte in Übereinstimmung mit der vorgesehenen Art der Anwendung durch den Patienten. Dazu wurde je eine Brausetablette in einem geeigneten Polypropylenmessbecher in 10 ml Wasser aufgelöst. Nach Abschluss der Brausereaktion wurde der Gehalt an gelöstem Budesonid mit einer HPLC-Methode bestimmt. Von jeder Brausetabletten-Rezeptur wurden insgesamt zwölf Bestimmungen durchgeführt. Parallel dazu wurde die Löslichkeit von reinem Budesonid in 10 ml Wasser ermittelt. Zur besseren Vergleichbarkeit wurde die ermittelte Wasserlöslichkeit von Budesonid auf 1,0 bzw. 100% normiert. Auf diese Weise ist der Einfluss der untersuchten Rezepturbestandteile auf die Löslichkeit von Budesonid deutlich zu erkennen. Die erhaltenen Löslichkeitswerte sind in Fig. 1 dargestellt.

Im Vergleich zur Löslichkeit von reinem Budesonid (siehe Fig. 1, Säule "Budesonid API") erhöht die Zugabe von 1,4% PVP in den Brausetabletten die Wasserlöslichkeit von Budesonid deutlich um ca. 20% (siehe Rezeptur G0397X414). Fehlt PVP (siehe Säule "G0397X416") bzw. fehlen PVP und Docusat-Natrium (siehe Säule "G0397X417") ist die freigesetzte Menge an Budesonid nur noch im Bereich des Kontrollansatzes oder sogar darunter. Eine Zugabe von 2,8% bzw. 4,8% PVP zur Brausetablettenrezeptur führte erneut zur einer Erhöhung der freigesetzten Menge an Budesonid (siehe Säulen "G0397X418 und G0397X419"). Das Zermörsern und Lösen von im Stand der Technik beschriebenen magensaftresistenten Kapseln (Fig. 1, rechte Säule) führt zu mangelhaften Ergebnissen.

### Beispiel 2

Der Zusatz von Docusat-Natrium verbessert die Anwendbarkeit einer Mundspüllösung von Budesonid weiter: Hierdurch wurde das hydrophobe Budesonid besser benetzbar gemacht und ein größerer Anteil des Budesonids kann in Lösung gehen (Tab. 2).

Die für den Patienten vorgesehene Anwendung sieht vor, dass der Patient die Brausetablette in einem Polypropylenmessbecher in 10 ml Wasser auflöst und anschließend zur Mundspülung anwendet. Das setzt jedoch voraus, dass der Inhalt des Messbechers annähernd quantitativ entnommen werden kann und keine nennenswerten Budesonid-Rückstände verbleiben (Tab. 2).

Zum Nachweis der vollständigen Entnahme wurde daher nach Auflösen einer Brausetablette die Mundspüllösung entnommen und der im Messbecher verbliebene Rückstand an Budesonid in 10 ml Methanol gelöst mit einer HPLC-Methode bestimmt. Von jeder Brausetablettenrezeptur wurden erneut 12 Bestimmungen durchgeführt. Die Tabelle 2 fasst die Ergebnisse des Versuchs zusammen. Dabei ist der Anteil der Budesonid-Dosierung angegeben, der nach Einnahme der Mundspüllösung im Messbecher haften bleibt und dem Patienten nicht zur Verfügung steht.

**Tab. 2 Nach Applikation verbleibender Rückstand von Budesonid im Messbecher**

| | **Brausetablette (Code)** | | | | | |
|---|---|---|---|---|---|---|
| | **G0397X414** | **G0397X416** | **G0397X419** | **G0397X415** | **G0397X418** | **G0397X417** |
| Budesonid-Rückstand im Messbecher | 5% | 6% | 12% | 13% | 13% | 25% |

Die Rezepturen mit Docusat-Natrium zeigen die eindeutig geringste Anhaftung an das Messbechermaterial (siehe Rezeptur Codes G0397X414 und G0397X416). Fehlt dieser Bestandteil in der Rezeptur nimmt der Anteil der Dosierung, der bei einer Applikation als Rückstand im Gefäß verbleibt, deutlich zu.

### Beispiel 3

Die erfindungsgemäße Brausetablette wurde zusätzlich durch die Addition von N-2,3-Trimethyl-2-isopropylbutanamid ("cooling agent") optimiert. Die Verwendung dieses Hilfsstoffes erzeugt eine kühlende Wirkung und ermöglicht dadurch eine verbesserte, angenehmere Anwendung als Mundspüllösung, die die Medikamententreue der Patienten erhöht.

Die Löslichkeit der in Tabelle 1 aufgeführten Zusammensetzungen wurde bestimmt und ist in Fig. 1 dargestellt. Die Zugabe von PVP und Docusat-Natrium erhöht die Löslichkeit einer Budesonid-Brausetablette um ca. 20%.

Mit der Bereitstellung dieser gebrauchsfertigen Mundspüllösung, basierend auf einer festen Brausetablette, liegt nun eine industriell herstellbare pharmazeutische Formulierung vor, die reproduzierbar zu dosieren und besonders zur Anwendung bei Entzündungen im oberen Verdauungstrakt geeignet ist.

### Beispiel 4

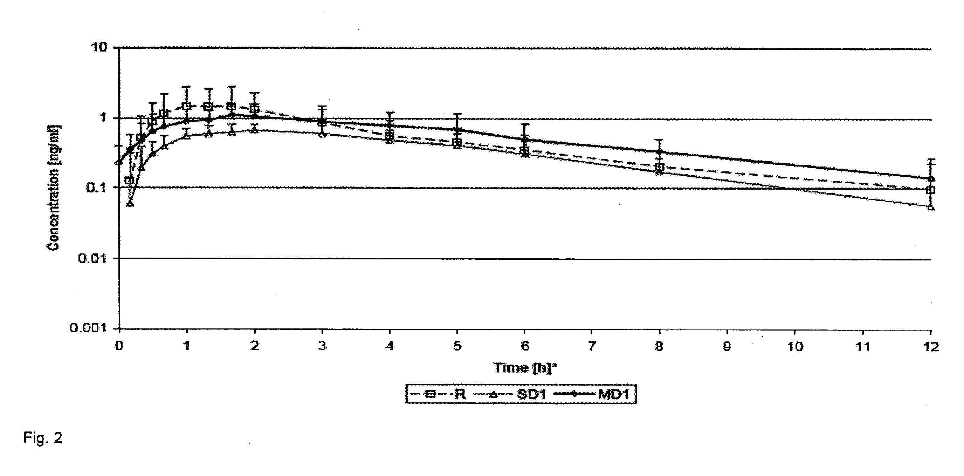

Die so hergestellte Formulierung (G0397X414) wurde *in vivo* an Patienten mit GVHD getestet und die Blutspiegel als Maß für die Verfügbarkeit von Budesonid an der Mukosa gemessen. Überraschenderweise wurde gefunden, dass bei der Anwendung der gewählten, optimierten Formulierung als Mundspüllösung ähnlich niedrige Blutspiegel generiert werden, wie bei der oralen Gabe (per os) einer gleichen Menge Budesonidlösung (Figur 2), obwohl im Mund resorbiertes Budesonid keinem first-pass-Effekt in der Leber unterliegt. Da im Magen/Darmtrakt resorbiertes Budesonid einem hohen First-Pass Effekt unterliegt, bei dem 90% des aufgenommenen Budesonids metabolisiert werden, zeigt diese vergleichbare Bioverfügbarkeit der Mundspüllösung einerseits eine effektive Konzentration an den betroffenen Mukosaschichten, sowie zusätzlich eine sichere, nebenwirkungsarme Anwendung, da keine höheren, sondern vergleichbar niedrige Blutspiegel gemessen werden. Tabelle 3 belegt diese Aussage durch die Vergleichbarkeit der pharmakologischen Daten der erfindungsgemäßen Formulierung mit verschiedenen oralen Darreichungsformen.

In einer klinischen Pilotstudie wurde die Wirksamkeit der erfindungsgemäßen Formulierung bei 18 Patienten mit oraler chronischer GvHD untersucht. Ziel der offenen, randomisierten Phase II Studie war es, den Schweregrad der oralen chronischen GvHD zu senken. Nach einer Anwendung der Budesonid-Brausetablette als Mundspüllösung über 8 Wochen konnte bei 11 von 18 Patienten (61 %) eine objektive Senkung des Schweregrades der oralen chronischen GvHD, gemessen mit dem modifizierten OMRS ("oral mucosa rating scale", nach Schubert et al, Cancer, 1992, Vol. 69, S. 2469-2477), erreicht werden. Als Definition für eine effektive Ansprechrate wurden nur solche Patienten gezählt, bei denen der modifizierte OMRS um mindestens 50 %, verglichen mit dem Ausgangswert, gesenkt werden konnte. Diese Pilotstudie belegt eindrucksvoll die Wirksamkeit der erfindungsgemäßen Formulierung.

Tab. 3 zeigt die Resorption von 3 mg Budesonid bei gesunden Probanden und Patienten nach einer Einmalgabe. Die Daten sind als Mittelwerte ± Standardabweichung bzw, als Median und der Streuung in Klammern angegeben.

**Tabelle 3**

| | Cmax | *tₘₐₓ* | *AUC₀₋ₜₗₐₛₜ* |
|---|---|---|---|
| | *(ng*/*mL)* | (h) | *(h*ng*/*mL)* |
| 7 Patienten, p.o.-Gabe der Mundspüllösung | 1.76 ± 1.38 | 1.7 (0.5-2.0) | 5.90 ± 4.42 |
| 12 Gesunde, p.o.-Gabe der Mundspüllösung | 1.23 ± 0.52 | 1.2 (0.3-1.7) | 2.67 ± 1.09 |
| 8 Gesunde, p.o.-Gabe einer magensaftresistenten Kapsel | 1.07 ± 0.63 | 5.0 (4.2-5.3) | 3.15 ± 2.00 |
| 7 Patienten, Anwendung als Mundspüllösung | 0.77 ± 0.23 | 2.0 (1.0-3.0) | 3.61 ± 1.32 |

| | | | |
|---|---|---|---|
| C_{max,} Spitzenspiegel im Plasma; tₘₐₓ, Zeitpunkt des Spitzenspiegels; AUC₀₋ₜₗₐₛₜ, Fläche unter der Plasma-Konzentrations-Zeit Kurve bis zur letzten messbaren Konzentration. | | | |

## Patentansprüche

1. Brausetablette enthaltend Budesonid zur Zubereitung einer oral verabreichbaren Mundspüllösung zur Anwendung in der Behandlung von entzündlichen Veränderungen des oberen Verdauungstraktes.

2. Brausetablette zur Anwendung nach Anspruch 1, **dadurch gekennzeichnet, dass** diese 0,1 bis 10 mg Budesonid pro Brausetablette enthält.

3. Brausetablette zur Anwendung nach einem der Ansprüche 1 oder 2, **dadurch gekennzeichnet, dass** diese Polyvinylpyrrolidon in einer Konzentration von 0,5 bis 10 Gew.-%, bezogen auf das Gewicht der Brausetablette, enthält.

4. Brausetablette zur Anwendung nach einem der vorhergehenden Ansprüche, **dadurch gekennzeichnet, dass** diese Docusat-Natrium in einer Konzentration von 0,1 ‰ bis 5,0 ‰ Gewicht, bezogen auf die Brausetablette, enthält.

5. Brausetablette zur Anwendung nach einem der vorhergehenden Ansprüche, **dadurch gekennzeichnet, dass** sie eine Brausemischung umfassend eine pharmazeutisch annehmbare Säure in fester Form und eine Carbonat und/oder Hydrogencarbonat enthaltende Verbindung umfasst.

6. Brausetablette zur Anwendung nach einem der vorhergehenden Ansprüche, **dadurch gekennzeichnet, dass** diese ein Mittel, das einen kühlenden Effekt im Mund bewirkt, in einer Konzentration von 0,1 bis 1,0 Gew.-%, bezogen auf die fertige Brausetablette, enthält.

7. Brausetablette zur Anwendung nach einem der vorhergehenden Ansprüche, **dadurch gekennzeichnet, dass** die entzündliche Veränderung des oberen Verdauungstraktes im Bereich der Mundhöhle und/oder des Pharynx lokalisiert ist.

8. Brausetablette zur Anwendung nach einem der Ansprüche 1-6, **dadurch gekennzeichnet, dass** es sich bei der entzündlichen Veränderung des oberen Verdauungstraktes um nicht-infektiöse Entzündungen handelt.

9. Brausetablette zur Anwendung nach einem der Ansprüche 1-6, **dadurch gekennzeichnet, dass** die entzündliche Veränderung des oberen Verdauungstraktes durch eine Chemotherapie hervorgerufen wurde.

10. Brausetablette zur Anwendung nach einem der Ansprüche 1-6, **dadurch gekennzeichnet, dass** die entzündliche Veränderung des oberen Verdauungstraktes verursacht wird durch Mukositis, eine Autoimmunerkrankung der Mundhöhle, Morbus Crohn im oberen Verdauungstrakt oder eosinophile Ösophagitis.

## Claims

1. An effervescent tablet containing budesonide for the formulation of a mouth rinse solution that can be orally administered for the use in the treatment of inflammatory changes of the upper digestive tract.

2. Effervescent tablet for the use according to claim 1, **characterized in that** it contains 0.1 to 10 mg budesonide per effervescent tablet.

3. Effervescent tablet for the use according to any one of claims 1 or 2, **characterized in that** it contains polyvinyl pyrrolidone in a concentration of from 0.5 to 10% by weight, based on the weight of the effervescent tablet.

4. Effervescent tablet for the use according to any one of the preceding claims, **characterized in that** it contains docusate sodium in a concentration of from 0.1 ‰ to 5.0 ‰ by weight, based on the effervescent tablet.

5. Effervescent tablet for the use according to any one of the preceding claims, **characterized in that** it comprises an effervescent blend comprising a pharmaceutically acceptable acid in solid form, and a carbonate and/or hydrogen carbonate containing compound.

6. Effervescent tablet for the use according to any one of the preceding claims, **characterized in that** it contains an agent causing a cooling effect in the mouth in a concentration of from 0.1 to 1.0% by weight, based on the finished effervescent tablet.

7. Effervescent tablet for the use according to any one of the preceding claims, **characterized in that** the inflammatory change of the upper digestive tract is localized in the area of the oral cavity and/or the pharynx.

8. Effervescent tablet for the use according to any one of claims 1 to 6, **characterized in that** the inflammatory change of the upper digestive tract are non-infectious inflammations.

9. Effervescent tablet for the use according to any one of claims 1 to 6, **characterized in that** the inflammatory change of the upper digestive tract was caused by chemotherapy.

10. Effervescent tablet for the use according to any one of claims 1 to 6, **characterized in that** the inflammatory change of the upper digestive tract is caused by mucositis, an autoimmune disease of the oral cavity, Crohn's disease in the upper digestive tract, or eosinophil oesophagitis.

## Revendications

1. Comprimé effervescent contenant du budésonide pour la préparation d'une solution de rinçage buccal administrable oralement, pour utilisation dans le traitement d'altérations inflammatoires du tractus digestif supérieur.

2. Comprimé effervescent pour utilisation selon la revendication 1, **caractérisé en ce que** celui-ci contient 0,1 à 10 mg de budésonide par comprimé effervescent.

3. Comprimé effervescent pour utilisation selon l'une des revendications 1 ou 2, **caractérisé en ce que** celui-ci contient de la polyvinylpyrrolidone à une concentration de 0,5 à 10% en poids par rapport au poids du comprimé effervescent.

4. Comprimé effervescent pour utilisation selon l'une des revendications précédentes, **caractérisé en ce que** celui-ci contient du docusate de sodium à une concentration de 0,1 ‰ à 5,0 ‰ en poids par rapport au comprimé effervescent.

5. Comprimé effervescent pour utilisation selon l'une des revendications précédentes, **caractérisé en ce qu'**il comporte un mélange effervescent comportant un acide pharmaceutiquement acceptable sous forme solide et un composé contenant du carbonate et/ou de l'hydrogénocarbonate.

6. Comprimé effervescent pour utilisation selon l'une des revendications précédentes, **caractérisé en ce que** celui-ci comporte un remède provoquant un effet rafraichissant dans la bouche, à une concentration de 0,1 à 1,0 % en poids par rapport au comprimé prêt à l'emploi.

7. Comprimé effervescent pour utilisation selon l'une des revendications précédentes, **caractérisé en ce que** l'altération inflammatoire du tractus digestif supérieur est localisée dans la zone de la cavité buccale et/ou du pharynx.

8. Comprimé effervescent pour utilisation selon l'une des revendications 1 à 6, **caractérisé en ce qu'**il ne s'agit pas d'inflammations infectieuses dans le cas de l'altération inflammatoire du tractus digestif supérieur.

9. Comprimé effervescent pour utilisation selon l'une des revendications 1 à 6, **caractérisé en ce que** l'altération inflammatoire du tractus digestif supérieur a été provoquée par une chimiothérapie.

10. Comprimé effervescent pour utilisation selon l'une des revendications 1 à 6, **caractérisé en ce que** l'altération inflammatoire du tractus digestif supérieur est provoquée par une mucosite, une maladie auto-immune de la cavité buccale, une maladie de Crohn dans le tractus digestif supérieur ou une oesophagite à éosinophiles.
